# EUROPEAN PATENT SPECIFICATION

(11) **EP 1 610 128 B1**
(45) Date of publication and mention of the grant of the patent: **01.08.2007**
(21) Application number: 04724781.2
(22) Date of filing: 31.03.2004
(51) Int. Cl.: G01N 33/543, G01N 33/53

(54) **IMMUNO-NEPHELOMETRY OF LIPOPROTEIN (a) AND REAGENT THEREFOR**
IMMUNNEPHELOMETRIE VON LIPOPROTEIN (A) UND REAGENS DAFÜR
IMMUNONEPHELOMETRIE DE LIPOPROTEINE (A) ET REACTIF ASSOCIE

(30) Priority: 31.03.2003 JP 2003094059
(43) Date of publication of application: 28.12.2005
(73) Proprietor: DENKA SEIKEN Co., Ltd., Tokyo 103-0025 (JP)
(72) Inventor: YAMAZAKI, Tadashi, c/o Denka Seiken Co., Ltd., Gosen-shi, Niigata 959-1695 (JP); GOTO, Hiromi, c/o Denka Seiken Co., Ltd., Gosen-shi, Niigata 959-1695 (JP)
(74) Representative: HOFFMANN EITLE
(86) International application number: PCT/JP2004/004606
(87) International publication number: WO 2004/088317

(56) References cited:
- JP-A- 10 073 595
- JP-A- 11 108 929
- JP-A- 2002 048 796
- US-A- 4 672 045
- ABE A ET AL: "Fully mechanized latex immunoassay for serum lipoprotein(a)." CLINICA CHIMICA ACTA; INTERNATIONAL JOURNAL OF CLINICAL CHEMISTRY. MAR 1994, vol. 225, no. 2, March 1994 (1994-03), pages 105-113, XP002372915 ISSN: 0009-8981

## Description

### Technical Field

The present invention relates to a method for quantitatively measuring an antigen having diverse phenotypes with accuracy in immunoassay.

### Background Art

In immunoassay, the quantitative determination on a molecular basis of an antigen having diverse phenotypes each differing in its molecular weight has required enzyme-linked immunoassay (ELISA) using several monoclonal antibodies that recognize a different antigenic site. For example, lipoprotein(a), a protein in blood, has a structure where apo(a), one of apoproteins, is bound with LDL. Apo(a) has repeats of domain structures exhibiting a high homology with plasminogen kringle 4. Because the number of this repeat varies according to an individual, the molecular weight of apo(a) is diversified and Lp(a) (lipoprotein(a)) is allowed to have a variety of phenotypes. For example, Utermann, G., et. al. have reported that lipoprotein(a) is broadly divided into 6 phenotypes (Utermann G. et al., J Coin Invest 1987; 80: 458-465). Therefore, when a molecule of a phenotype with a certain molecular weight is used as a reference material to measure molecules of other phenotypes with molecular weights different therefrom in measurement on a weight basis commonly used in immunoassay, a value that deviates from a measurement value measured on a molecular basis is obtained because of difference in the molecular weight of each phenotype. In this case, there is also a problem that phenotypic differences result in difference in reactivity with a certain antibody. For quantitatively measuring molecules of all phenotypes with accuracy regardless of phenotypic differences, ideal is not measurement on a weight concentration basis generally used for measuring proteins in serum but measurement on a molecular basis by molar concentration. Theoretically, enzyme-linked immunoassay using a monoclonal antibody that reacts in a one-to-one relationship with an antigen is desirable for performing measurement on a molecular basis using immunoassay, and the use of several monoclonal antibodies is required for dealing with diverse phenotypes in the measurement (Clin Chem 1995; 41: 246-255). On the other hand, in the measurement of lipoprotein(a), turbidimetric immunoassay using a polyclonal antibody is also widely used, which generally aims at measurement on a weight concentration basis. Therefore, the turbidimetric immunoassay has presented a problem of a measurement value that deviates from those obtained by enzyme-linked immunoassay due to the phenotypic differences of an antigen, (Curr Cardiol Rep 1999; 1: 105-111).

Abe A. et al.; Clinica Chimica Acta; International Journal of Clinical Chemistry, March 1994, vol. 225, no. 2, pp. 105-113, relates to a latex enhanced turbidimetric immunoassay of lipoprotein(a). There is no indication in this document that a basic amino acid is added to the assay system.

US 4,672,045 A relates to an immunoassay based on an antibody antigen interaction. Column 3 discloses that latex agglutination with near infrared turbidimetry may be used (lines 18 to 23). This document does not disclose an antigen antibody reaction of lipoproteins having several phenotypes. Furthermore, it does not specifically indicate that a basic amino acid is added to the assay system.

Lipoprotein(a) is associated with arteriosclerosis, ischemic heart disease, and so on. The concentration of lipoprotein(a) in blood can be used in the assessment of morbidity risk for these diseases. Under such circumstances, a method capable of rapidly and conveniently measuring the accurate concentration of lipoprotein has been desired.

### Disclosure of the Invention

An object of the present invention is to solve a problem of a measurement value obtained by turbidimetric immunoassay that deviates from those obtained by enzyme-linked immunoassay due to the phenotypic differences of an antigen. Specifically, the present invention is intended to provide a method of bringing a measurement value obtained by turbidimetric immunoassay into agreement with those obtained by enzyme-linked immunoassay by the adjustment of reagent components.

The present inventors have found that the adjustment of reagent components allows the control of a measurement value in turbidimetric immunoassay for measuring an antigen having several phenotypes. Namely, the present inventors have completed the present invention by finding that a large amount of an antibody and a given amount of a basic amino acid such as arginine are added to reagent components, thereby circumventing the influence of variations in measurement values attributable to phenotypic differences and obtaining a measurement value having a high correlation with a measurement value obtained by enzyme-linked immunoassay that is capable of measurement on a molecular basis.

That is, the present invention is as follows:
[1] Latex turbidimetric immunoassay using an antigen-antibody reaction of lipoprotein(a) having several phenotypes, wherein, in detection utilizing the immunoassay, the amount of an antibody against the lipoprotein(a) added to an assay system is adjusted and a basic amino acid is added to the assay system, thereby circumventing the variability of a measurement value attributable to phenotype variety and obtaining a measurement value having a high correlation with a measurement value of the lipoprotein(a) in a biological sample that is measured on a molecular basis;
[2] The immunoassay of [1], wherein the amount of the antibody added is greater than or equal to 0.16 mg/mL in a reaction solution at the time of the antigen-antibody reaction;
[3] The immunoassay of [2], wherein the amount of the antibody added is from 0.16 mg/mL to 0.23 mg/mL inclusive in the reaction solution at the time of the antigen-antibody reaction;
[4] The immunoassay of any of [1] to [3], wherein the amount of the basic amino acid added is greater than or equal to 15% by weight in the reaction solution at the time of the antigen-antibody reaction;
[5] The immunoassay of [4], wherein the amount of the basic amino acid added is from 15% by weight to 17% by weight inclusive in the reaction solution at the time of the antigen-antibody reaction;
[6] The immunoassay of any of [1] to [5], wherein the basic amino acid is arginine;
[7] A detection reagent for latex turbidimetric immunoassay using an antigen-antibody reaction of lipoprotein(a) having phenotypes, the reagent comprising: an antibody against the lipoprotein(a) in such an amount that the amount of the antibody is greater than or equal to 0.16 mg/mL in a reaction solution at the time of the antigen-antibody reaction; and a basic amino acid in such an amount that the amount of the basic amino acid is greater than or equal to 15% by weight in the reaction solution at the time of the antigen-antibody reaction, wherein the latex turbidimetric immunoassay circumvents the variability of a measurement value attributable to phenotype variety and obtains a measurement value having a high correlation with a measurement value of the lipoprotein(a) in a biological sample that is measured on a molecular basis;
[8] The detection reagent for latex turbidimetric immunoassay of [7], wherein the amount of the antibody added is from 0.16 mg/mL to 0.23 mg/mL inclusive in the reaction solution at the time of the antigen-antibody reaction;
[9] The detection reagent for latex turbidimetric immunoassay of [7] or [8], wherein the amount of the basic amino acid added is from 15% by weight to 17% by weight inclusive in the reaction solution at the time of the antigen-antibody reaction; and
[10] The detection reagent for latex turbidimetric immunoassay of any of [7] to [9], wherein the basic amino acid is arginine.

The present specification encompasses contents described in the specifications and/or drawings of Japanese Patent Application No. 2003-094059 that serves as a basis for the priority of the present application.

### Brief Description of the Drawings

Figure is a diagram showing the correlation between measurement values obtained by control assay and measurement values obtained by a latex agglutination method in each concentration of an antibody;
Figure 2 is a diagram showing the deviation of measurement values of samples of each phenotype from a regression line in each concentration of an antibody;
Figure 3 is a diagram showing the correlation between measurement values obtained by control assay and measurement values obtained by a latex agglutination method in each concentration of arginine; and
Figure 4 is a diagram showing the deviation of measurement values of samples of each phenotype from a regression line in each concentration of arginine.

### Best Mode for Carrying Out the Invention

Hereinafter, the present invention will be described in detail.

The present invention is intended to a method of circumventing the variability of a measurement value attributable to the phenotypic differences of an antigen in the detection, using turbidimetric immunoassay, of the antigen having diverse phenotypes. The antigen to be detected is not limited as long as it is a protein having several phenotypes. A protein having a varying molecular weight attributable to phenotypic differences is preferable, with lipoprotein(a) particularly preferred. It is known that lipoprotein(a) has 6 phenotypes designated as F, B, S1, S2, S3, and S4 (Nippon Rinsho in Japanese (Japanese Journal of Clinical Medicine) 1999; 57 Suppl: 42-44). These phenotypes can be determined by the method of Utermann et al. (Utermann, G. et al., Proc. Natl. Acad. Sci. USA 1989; 86: 4171-4174): the phenotypes are classified according to their relative mobility compared to the mobility of apo-B on SDS-PAGE, as F (faster, with a molecular weight smaller than that of apo-B), B (equal), and S1, S2, S3 and S4 (all slower, in descending order of mobility).

Turbidimetric immunoassay is a method in which an antibody contained in an assay reagent is subjected to antigen-antibody reaction with an antigen in a biological sample to form an aggregate whose formation is measured by absorbance to qualitatively or quantitatively determine the antigen in the biological sample. For example, the antibody used in the turbidimetric immunoassay is sensitized (bound) to an insoluble carrier. No limitation is imposed on the carrier used, and, for example, latex particles, bentonite, collodion, kaoline, and immobilized sheep erythrocytes can be used. The latex particles are preferred. Turbidimetric immunoassay using latex particles is called latex turbidimetric immunoassay, in which the latex particles sensitized (bound) with an antibody is mixed with an antigen in a biological sample to be measured, to form the agglutination of the latex particles whose degree is measured by absorbance to qualitatively or quantitatively determine the antigen. The biological sample used in the present invention is not limited, and blood, serum, and plasma can preferably be used.

Examples of the latex particles include polystyrene latex particles of homopolymers and/or copolymers of vinyl monomers such as vinyl chloride, acrylonitrile, vinyl acetate, acrylic ester, and methacrylic ester, butadiene-based copolymer latex particles such as styrene-butadiene copolymers and methyl methacrylate-butadiene copolymers, and polyvinyltoluene latex particles. Among them, polystyrenic latex particles are preferred in that they have the excellent property of adsorbing, for example, a variety of proteins or polypeptides and can stably maintain biological activity for a long period. The above-described latex particles have a particle size of preferably 0.01 to 1 µm, more preferably 0.1 to 1 µm. The latex particles having a particle size less than 0.01 µm lead to frequent occurrence of fine agglutination and ununiformity of an apparent particle size and adversely affect simultaneous reproducibility and so on. Moreover, in some cases, these latex particles are not agglutinated sufficiently for the number of antibodies. If a particle size is larger than 1 µm, autoagglutination is promoted and dispersibility is reduced.

The antibody used in sensitization of the latex particles is preferably a polyclonal antibody from a rabbit, a goat, a sheep, a pig, a horse, or the like. The polyclonal antibody can be obtained by immunizing an animal with purified lipoprotein(a) that serves as an immunogen to give antiserum, from which the polyclonal antibody is then purified by an appropriately selected method or combined methods from methods known in the art such as ammonium sulfate precipitation, ion exchange chromatography that employs an anion exchanger such as DEAE cellulose, molecular sieve chromatography that conducts separation according to molecular sizes or shapes, hydroxyapatite chromatography, and affinity chromatography. No limitation is imposed on the phenotype of the lipoprotein(a) used in this immunization. For the lipoprotein(a), fractions with a specific gravity of 1.063 to 1.15 can be obtained from, for example, normal human serum by ultracentrifugation. The fraction can be purified by chromatography that uses a column of Sepharose CL-4B and so on.

A method of sensitizing the latex particles with the antibody is not particularly limited. For example, the antibody may be adsorbed physically or bound chemically to the carrier. To be more specific, the carrier can be sensitized with the antibody by mixing the antibody with the carrier, followed by heating and shaking at 30 to 37°C for 1 or 2 hour(s). The amount of the antibody with which the carrier is sensitized can appropriately be set according to the particle size of the carrier used. It is preferred that unsensitized sites on the surface of the carrier should be blocked with bovine serum albumin, human serum albumin, rabbit serum albumin, ovalbumin, or the like, after the carrier is sensitized with the antibody. It is preferred that the carrier sensitized with the polyclonal antibody should be stored as a medium dispersion until it is reacted with the biological sample. For example, a phosphate buffer, a glycine buffer and the like can be used as a medium for dispersing the carrier. The content of the carrier sensitized with the polyclonal antibody can be typically 0.05 to 0.5% by weight, preferably 0.1 to 0.3% by weight, relative to the amount of the medium dispersion. Bovine serum albumin, gelatin, gum arabic, or the like may be added to the medium, if necessary.

Latex turbidimetric immunoassay can be conducted by mixing antibody-sensitized latex particles with a biological sample in an appropriate buffer. In the present invention, a larger amount of the antibody and a basic amino acid is allowed to exist in a reaction system when the antibody-sensitized latex particles are mixed with the biological sample and agglutinated. The final concentration of an antibody added to a reaction system is 0.05 to 0.1 mg/mL in typical turbidimetric immunoassay, whereas in the present invention, the antibody is added at a concentration greater than or equal to at least 0.15 mg/mL, preferably greater than or equal to 0.16 mg. An upper limit thereto is not limited in light of the effect of suppressing the variability of a measurement value attributable to phenotype variety. However, the concentration is preferably 1 mg/mL or less, more preferably 0.3 mg/mL or less, particularly preferably 0.23 mg/mL or less, from the viewpoint of obtaining a favorable image of agglutination in the latex turbidimetric immunoassay. For adjusting the amount of the antibody added, the amount of the antibody-sensitized latex particles added to the reaction system may be increased, or the amount, per unit latex particle, of the antibody used for sensitization of the latex particles may be increased. Alternatively, the antibody-sensitized latex particles, which are prepared as sensitized latex particles at a concentration of 0.05 to 0.5% by weight relative to the medium dispersion as described above, may be allowed to have a higher concentration in the medium dispersion.

Examples of the basic amino acid include arginine, histidine, and citrulline. Among them, arginine is particularly preferred. The amount of the basic amino acid added is greater than or equal to 12%, preferably greater than or equal to 15%, in terms of its final concentration in the reaction system when the antibody-sensitized latex particles are mixed with the biological sample and agglutinated. An upper limit of the concentration of the basic amino acid is not limited in light of the effect of suppressing the variability of a measurement value attributable to phenotype variety. For example, the basic amino acid can be used at a concentration on the order of 40%. However, the concentration is preferably 25% or less, more preferably 20% or less, particularly preferably 17% or less, from the viewpoint of obtaining a favorable image of agglutination, although arginine.

A latex agglutination method according to the present invention is typically performed by mixing 1 volume of a biological sample with 40 volumes of a first reagent containing a buffer and 20 volumes of a sensitized latex particle solution (second reagent). However, any ratio at which abundance ratios of an antigen in the biological sample and a sensitized latex particle are proper and a favorable image of agglutination is obtained may be used without being limited to this mixing ratio. Alternatively, the first reagent and the second reagent may be used in a single solution. The concentration of the particle in the sensitized latex particle solution may appropriately be adjusted according to a ratio of the volume of the sensitized latex particle solution added to a reaction system to the total volume of the reaction system. For example, a phosphate buffer, pH 7, or glycine buffer, pH 7, are used as the buffer constituting the first reagent. A basic amino acid may be added to the first reagent, the second reagent, or both, as long as its final concentration falls in the above-described concentration range. In the typical example described above, the preferred concentration of the antibody in the sensitized latex particle solution is from 0.5 mg/mL to 0.7 mg/mL inclusive. For example, when the basic amino acid is added to both of the first reagent and the second reagent, the basic amino acid may be added at a concentration of 7 to 10% for the first reagent and 30% for the second reagent. The preferred concentration of the antibody or the basic amino acid in these reagents can readily be determined from the final concentration in the final reaction system and the volume ratio of each of the reagents.

Reaction is performed by mixing the biological sample with the first reagent and the second reagent. Although the order of mixing is not limited, the biological sample may be mixed initially with the first reagent with stirring for a several minutes, preferably 1 to 5 minute(s), more preferably 5 minutes, and subsequently mixed with the second reagent with stirring. Agglutination reaction is performed for 1 to 4 minute(s) to examine the degree of agglutination. A temperature at which the above-described procedures are performed is not limited, but preferably 37°C. These procedures can also be performed within a plastic cell or a glass cell. In this case, the cell is irradiated from the outside with light from visible light to light in the near-infrared region, for example, a light having a wavelength of usually 400 to 2400 nm, preferably 550 to 800 nm. A change in absorbance or a change in scattering light intensity is then detected to measure the degree of agglutination of the carrier particles. In this case, the use of a calibration curve prepared in advance allows the calculation of the amount (concentration) of the lipoprotein(a) in the sample. The phenotype of the lipoprotein(a) that is used for preparing this calibration curve is not limited. The agglutination can be measured using, for example, a fully-automiated latex agglutination measurement apparatus LPIA-S500 (Mitsubishi Chemical), an automated analyzer TBA-200FR (Toshiba), and a Hitachi 7170 automated analyzer (Hitachi).

The agglutination reaction may be conducted in a solution such as a physiological salt solution or an appropriate buffer (pH 5.0 to 10), for example, a phosphate buffer, a borate buffer, a Tris buffer and the like.

According to the method of the present invention, the variability of a measurement value attributable to phenotype variety is circumvented, and a measurement value having a high correlation with a measurement value of lipoprotein(a) in a biological sample that is measured on a molecular basis is obtained. The "measurement value of lipoprotein(a) in a biological sample that is measured on a molecular basis" used herein refers to, for example, a value measured by enzyme-linked immunoassay (ELISA) conducted under particular conditions.

The ELISA (Enzyme Linked Immuno-Sorbent Assay) method is a method in which an antibody is labeled with an enzyme and a substance (antigen) that binds to the antibody is detected. Especially, the ELISA method is widely used as a method of detecting an antigen protein and as an analysis method of using antigen-antibody reaction to detect an antigen protein in a sample or conversely, an antibody that binds to a particular antigen protein. The ELISA method detects an antibody reacted with an antigen to be measured, using a second antibody chemically bound in advance with an enzyme such as peroxidase or galactosidase, and detects the presence or absence or the amount of antigen of interest on the basis of the degree of color developed by adding, to a reaction system, a substrate that develops a color through enzyme reaction.

The general mainstream of the detection of a protein of interest in the ELISA method is detection by an antibody-based sandwich method in which an antigen is sandwiched between an immobilized antibody and a labeled antibody using the combination of a polyclonal antibody and a polyclonal antibody, a monoclonal antibody and a monoclonal antibody, or a polyclonal antibody and a monoclonal antibody ("Seikagaku Jikkenho in Japanese (Experiments in Biochemistry) 11 - Immunoassay," published by Tokyo Kagaku Dozin, Nov. 15, 1989).

In the ELISA method, the binding of one molecule of the immobilized antibody and one molecule of the antigen-one molecule of the labeled antibody is generally established. Unless several epitopes (antigenic determinants) recognized by the labeled antibody are present on one antigen molecule, the above-described binding is established even for any phenotype of an antigen having diverse phenotypes, so that measurement on a molecular basis is made possible. That is, even when a molecule of any phenotype is used as a reference material, molecules of the other phenotypes can accurately be measured on a molecular basis.

For example, in lipoprotein(a), a molecule of apo(a), one of structural proteins, has repeats of structures called kringle 4, which are divided into 10 different types from type 1 to type 10. Of these types, kringle 4 type 1 and type 3 to type 10 are present as a single copy in all apo(a) species, whereas kringle 4 type 2 is present in a variable number of repeats in each apo(a) molecule, allegedly varying from 3 to 40. Namely, variations in a molecular weight attributable to the phenotypic differences of lipoprotein mainly result from the number of kringle 4 type 2 repeats. This indicates that accurate measurement on a molecular basis can not be conducted in the measurement using ELISA, of lipoprotein(a) from the number of the repeat of each kringle 4 type, if an antibody against kringle 4 type 2 is contained in both immobilized and labeled antibodies (Clin Chem 1995; 41: 245-255). Moreover, identical or similar epitopes are present on domains of different kringle 4 types. This rather allows measurement on a molecular basis, because the binding of one molecule of the immobilized antibody-one molecule of the antigen-one molecule of the labeled antibody is established without exception when the immobilized antibody and the labeled antibody are selected from among antibodies capable of binding to any of kringle 4 type 1 and kringle 4 type 3 to type 10 and recognizing an epitope that is not commonly present in different kringle 4 types and the immobilized antibody and the labeled antibody bind to a separate kringle 4 type. An antibody that recognizes an epitope present in a domain other than the kringle 4 domain and present in a single copy in apo(a) may be used as at least the labeled antibody, preferably both of the immobilized antibody and the labeled antibody. In the present invention, the "measurement value of lipoprotein(a) that is measured on a molecular basis" refers to a measurement value obtained by ELISA conducted under such a condition that the binding of one molecule of the immobilized antibody-one molecule of the antigen-one molecule of the labeled antibody is established. Examples of such ELISA include ELISA described in Clin Chem 1995; 41: 246-255. When a measurement value obtained by such an ELISA method is correlated with the measurement value obtained by the method of the present invention, its correlation coefficient (R²) is 0.97 or more, preferably 0.98 or more, still more preferably 0.99 or more.

The turbidimetric immunoassay of the present invention that circumvents the variability of a measurement value attributable to phenotype variety and obtains a measurement value having a high correlation with a measurement value of lipoprotein(a) in a biological sample that is measured on a molecular basis is turbidimetric immunoassay in which a calibration curve prepared by the same measurement as below using any lipoprotein(a) phenotype as sample substantially shows parallelism with a calibration curve prepared by plotting the relationship of measurement values expressed as theoretical concentrations and absorbance variations in the measurement by the turbidimetric immunoassay, of a preparation obtained by using a lipoprotein(a) reference material (PRM; IFCC proposed reference material) certified by IFCC (International Federation of Clinical Chemistry and Laboratory Medicine, Via Carlo Farini 81, 20159 Milano, Italy) as a sample that is in turn subjected to serial dilution with a physiological saline.

The method of the present invention circumvents the variability of a measurement value attributable to phenotype variety and obtains a measurement value having a high correlation with a measurement value of lipoprotein(a) in a biological sample that is measured on a molecular basis. This means that a nearly identical measurement value is obtained at all times even if the phenotype of a molecule used as a reference material is changed. Thus, the method of the present invention is a method that obtains a constant measurement value, independently of the phenotype of a molecule used as a reference material.

Hereinafter, the present invention will be described specifically with reference to Examples. However, the present invention is not intended to be limited to Examples below.

### [Example 1]

Change in measurement value obtained using reagents having varying concentrations of latex (amounts of antibody) (correlation with control method (ELISA))

A rabbit anti-human lipoprotein(a) polyclonal antibody (available from DAKO) was mixed with latex particles (available from Sekisui Chemical) and heated at room temperature for 60 minutes and subsequently in a thermostat bath at 60°C for 50 minutes, followed by cooling in cold water for 20 minutes to thereby conduct sensitization. The polyclonal antibody was sensitized in an amount of 0.14 mg per mg of the latex particles. The sensitized latex particles were dispersed at a concentration of 0.5% by weight in 0.17 M glycine buffer, pH 7, to give a rabbit anti-human lipoprotein polyclonal antibody dispersion.

Dispersed suspensions of the latex particles sensitized with the rabbit anti-human lipoprotein(a) polyclonal antibody were prepared so that the final concentrations of the antibody in second reagents were brought to approximately 0.3, 0.4, 0.5, and 0.7 mg/mL, respectively. The dispersed suspensions were used as the second reagents. A glycine buffer supplemented with 0.1 M NaCl, 0.05 M EDTA, 1% BSA, and 25 mg/mL normal rabbit globulin for the purpose of preventing nonspecific reaction was used as a first reagent. Arginine as a basic amino acid was added at a concentration of 10% for the first reagent and 30% for the second reagent. The final concentration of the arginine in a reaction solution was 16.7%.

Twenty human serum samples respectively containing a different lipoprotein(a) phenotype were used as samples to be measured. Among 20 samples, 4 samples each for each of phenotypes B, S1, S3, S4, and S5 were used (available from Technoclone).

For performing reaction, 160 µL of the first reagent is added to 4 µL of each serum sample and stirred. After five minutes, 80 µL of each second reagent was added and stirred to conduct agglutination reaction. A Hitachi 7170 automated analyzer was used in measurement and set to measure the agglutination reaction at 37°C for 4 minutes from about 1 minute into the reaction as absorbance variations at a wavelength of 570 nm. For calculating concentrations from the absorbance variations, a calibration curve showing the relationship between the concentration and the absorbance variations was prepared in advance by using a reference material (available from Technoclone) having a known concentration as a sample and measuring it under the same condition.

The above-described 20 samples were measured as follows by enzyme-linked immunoassay that serves as a control method. A monoclonal antibody used can be obtained by a method described in Clin Chem 1995; 41: 246-255. An anti-human lipoprotein(a) monoclonal antibody (which binds to Kringle 4 type 2 but does not bind to kringle 4 type 1 and type 3 to type 10) was immobilized at 0.5 µg/well in 96-well microtiter plate manufactured by Nunc (100 µL of 5 µg/mL antibody solution in 0.1 M sodium bicarbonate buffer (pH 9.6) was placed in the well and stirred at room temperature for 1 hour, followed by overnight incubation at 4°C). The wells were washed three times with PBS, pH 7.4. The plate was blocked by the addition of 300 µL of PBS containing 30 g/L BSA to the wells and one-hour incubation at room temperature. Following blocking, the wells were washed three times with PBS, pH 7.4. Then, 100 µL of each of the above-described samples was added to the wells and stirred at 28°C for 1 hour. Before the addition, the samples were appropriately diluted with PBS containing 1 g/L BSA and 0.5 mL/L Tween 20. After the wells were washed three times with PBS, pH 7.4, 100 µL of a HRP-labeled anti-human lipoprotein(a) monoclonal antibody (which binds to a kringle 4 domain other than kringle 4 type 2 domain) solution was added and stirred at 28°C for 1 hour. Subsequently, color reaction was performed by adding OPD and H₂O₂. After 15 minutes, the reaction was stopped by adding, 100 µL of 1 mol/L sulfuric acid. The absorbance of the reaction solutions after the termination of the reaction was measured at 495 nm. For calculating concentrations from the absorbance variations, a calibration curve showing the relationship between the concentration and the absorbance variations was prepared in advance by using a reference material having a known concentration as a sample and measuring it under the same condition.

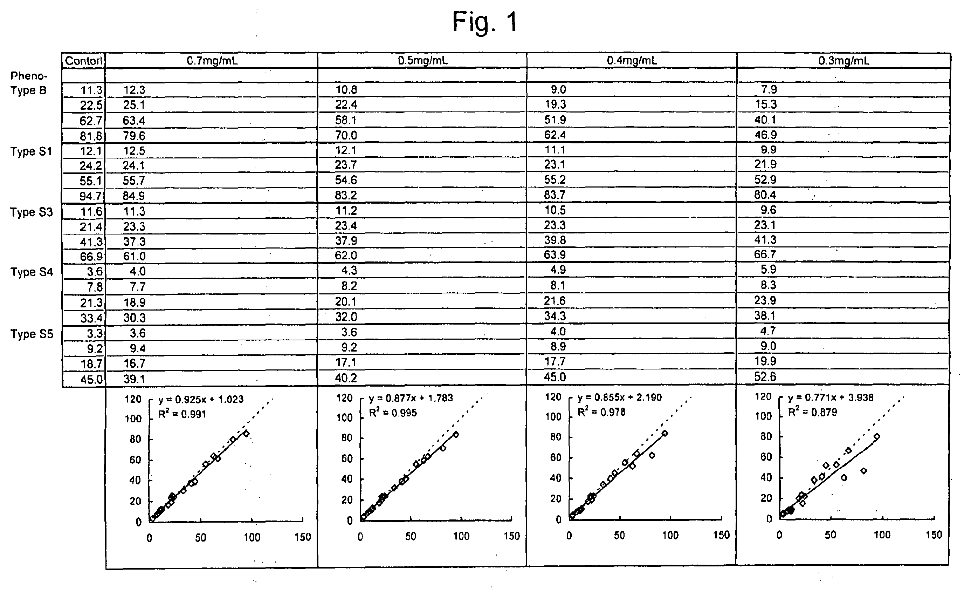

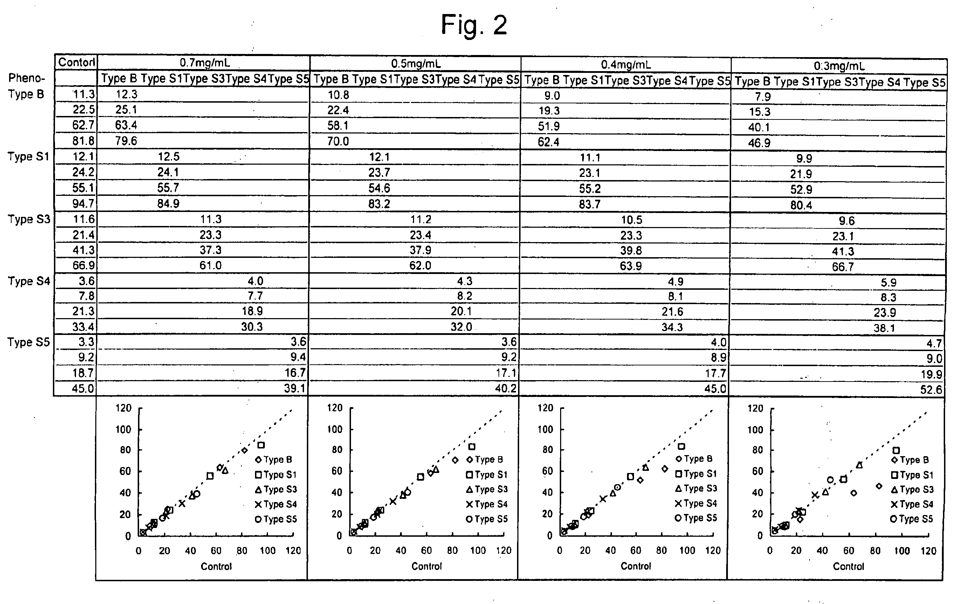

As a result, the samples having measurement values that deviate from a regression line determined by a correlation with enzyme-linked immunoassay were observed in the measurement with the reagents where the concentrations of the antibody in the second reagents were 0.3 mg/mL and 0.4 mg/mL, respectively, whereas no sample having a measurement value that deviate from the regression line determined by a correlation with enzyme-linked immunoassay was observed in the measurement with the reagents where the concentrations of the antibody in the second reagents were 0.5 mg/mL and 0.6 mg/mL, respectively. Figure 1 shows the correlation between the measurement values obtained by the control assay and the measurement values obtained by the latex agglutination method in each concentration of the antibody. Figure 2 shows the deviation of the measurement values of the samples of each phenotype from the regression line.

It has been confirmed the adjustment of the concentration of an antibody in a reagent allows agreement with a measurement value obtained by enzyme-linked immunoassay that serves as a control.

### [Example 2]

Change in measurement value obtained using reagents having varying concentrations of arginine (correlation with control method (ELISA))

A dispersed suspension of latex particles sensitized with a rabbit anti-human lipoprotein(a) polyclonal antibody was prepared in the same way as Example 1.

The dispersed suspension of the latex particles sensitized with the rabbit anti-human lipoprotein(a) polyclonal antibody was prepared so that the final concentration of the antibody in a second reagent was brought to approximately 0.7 mg/mL. Arginine as a basic amino acid was added to the first reagent so that the final concentrations of arginine in reaction solutions were brought to 10%, 15, and 17%, respectively (the concentration in the second reagent is 30%).

Measurement conditions and samples to be measured were the same as Example 1.

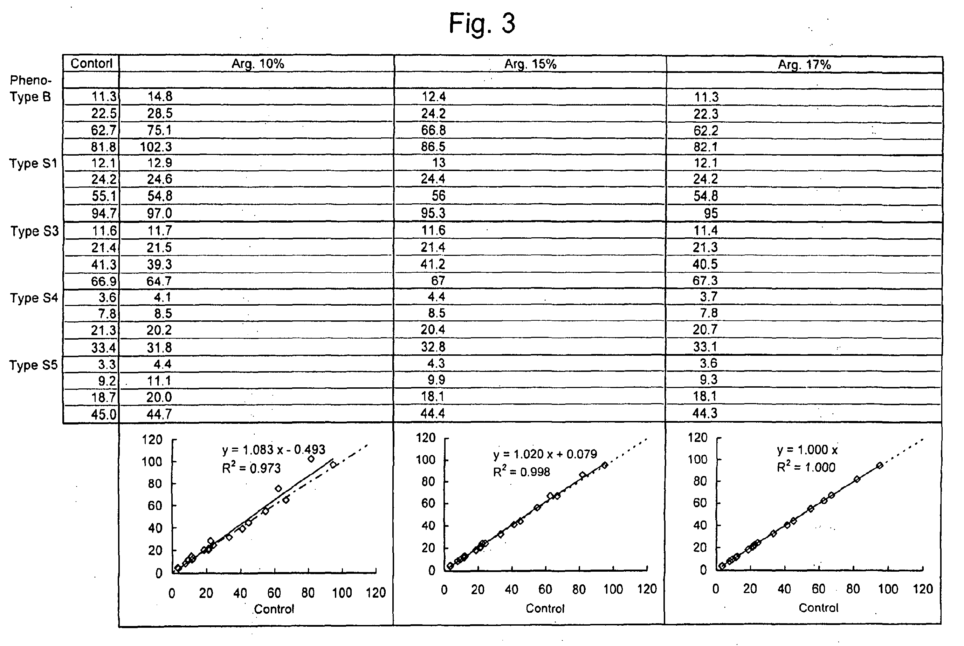

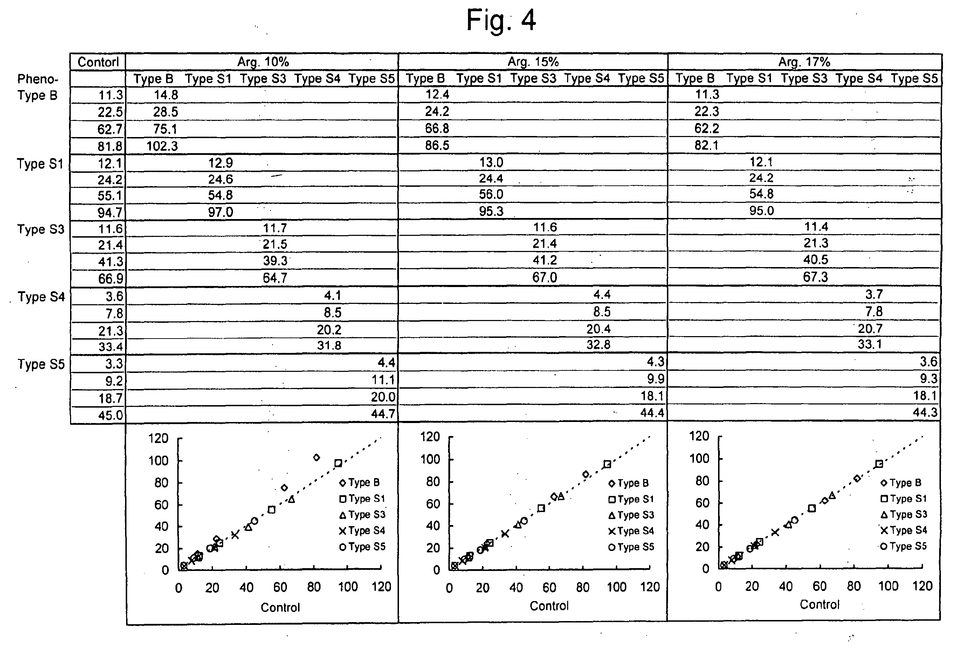

As a result, the samples having measurement values that deviate from a regression line determined by a correlation with enzyme-linked immunoassay were observed when the reaction solution having the concentration of arginine of 10% was used, whereas no sample having a measurement value that deviate from the regression line determined by a correlation with enzyme-linked immunoassay was observed when the reaction solutions having the concentrations of arginine of 15% and 17%, respectively, were used. Figure 3 shows the correlation between the measurement values obtained by the control assay and the measurement values obtained by the latex agglutination method in each concentration of the arginine. Figure 4 shows the deviation of the measurement values of the samples of each phenotype from the regression line.

It has been confirmed the adjustment of the concentration of arginine in a reagent allows agreement with a measurement value obtained by enzyme-linked immunoassay.

### Industrial Applicability

As described in Examples, latex turbidimetric immunoassay can circumvent the variability of a measurement value attributable to the phenotypic differences of human lipoprotein(a) having diverse phenotypes by increasing the amount of an antibody added to a reaction system and adding a basic amino acid having a given concentration to the reaction system.

The present invention allows rapid and convenient measurement with an automated analyzer, which gives a measurement value having an excellent correlation with a measurement value obtained by enzyme-linked immunoassay, when an antigen having diverse phenotypes is measured.

## Claims

1. Latex turbidimetric immunoassay using an antigen-antibody reaction of lipoprotein(a) having several phenotypes, wherein, in detection utilizing the immunoassay, the amount of an antibody against the lipoprotein(a) added to an assay system is adjusted and a basic amino acid is added to the assay system, thereby circumventing the variability of a measurement value attributable to phenotype variety and obtaining a measurement value having a high correlation with a measurement value of the lipoprotein(a) in a biological sample that is measured on a molecular basis.

2. The immunoassay according to claim 1, wherein the amount of the antibody added is greater than or equal to 0.16 mg/mL in a reaction solution at the time of the antigen-antibody reaction.

3. The immunoassay according to claim 2, wherein the amount of the antibody added is from 0.16 mg/mL to 0.23 mg/mL inclusive in the reaction solution at the time of the antigen-antibody reaction.

4. The immunoassay according to any one of claims 1 to 3, wherein the amount of the basic amino acid added is greater than or equal to 15% by weight in the reaction solution at the time of the antigen-antibody reaction.

5. The immunoassay according to claim 4, wherein the amount of the basic amino acid added is from 15% by weight to 17% by weight inclusive in the reaction solution at the time of the antigen-antibody reaction.

6. The immunoassay according to any one of claims 1 to 5, wherein the basic amino acid is arginine.

7. A detection reagent for latex turbidimetric immunoassay using an antigen-antibody reaction of lipoprotein(a) having phenotypes, the reagent comprising: an antibody against the lipoprotein(a) in such an amount that the amount of the antibody is greater than or equal to 0.16 mg/mL in a reaction solution at the time of the antigen-antibody reaction; and a basic amino acid in such an amount that the amount of the basic amino acid is greater than or equal to 15% by weight in the reaction solution at the time of the antigen-antibody reaction, wherein the Latex turbidimetric immunoassay circumvents the variability of a measurement value attributable to phenotype variety and obtains a measurement value having a high correlation with a measurement value of the lipoprotein(a) in a biological sample that is measured on a molecular basis.

8. The detection reagent for latex turbidimetric immunoassay according to claim 7, wherein the amount of the antibody added is from 0.16 mg/mL to 0.23 mg/mL inclusive in the reaction solution at the time of the antigen-antibody reaction.

9. The detection reagent for Latex turbidimetric immunoassay according to claim 7 or 8, wherein the amount of the basic amino acid added is from 15% by weight to 17% by weight inclusive in the reaction solution at the time of the antigen-antibody reaction.

10. The detection reagent for latex turbidimetric immunoassay according to any one of claims 7 to 9, wherein the basic amino acid is arginine.

## Patentansprüche

1. Latex-turbidometrischer Immunassay unter Verwendung einer Antigen-Antikörper-Reaktion eines Lipoproteins (a) mit etlichen Phänotypen, worin bei dem Nachweis unter Verwendung des Immunassays die Menge eines Antikörpers gegen das Lipoprotein (a), zugefügt zu einem Assaysystem, eingestellt wird und eine basische Aminosäure dem Assaysystem zugefügt wird, wodurch die Variabilität eines Meßwerts, der einer Phänotypvarietät zuzuschreiben ist, umgangen wird und ein Meßwert mit einer hohen Korrelation mit einem Meßwert des Lipoproteins (a) in einer biologischen Probe erhalten wird, gemessen auf einer molekularen Basis.

2. Immunassay gemäß Anspruch 1, wobei die Menge des zugefügten Antikörpers mehr als 0,16 mg/ml in einer Reaktionslösung zum Zeitpunkt der Antigen-Antikörper-Reaktion beträgt oder dem entspricht.

3. Immunassay gemäß Anspruch 2, wobei die Menge des zugefügten Antikörpers von 0,16 bis einschließlich 0,23 mg/ml in der Reaktionslösung zum Zeitpunkt der Antigen-Antikörper-Reaktion beträgt.

4. Immunassay gemäß einem der Ansprüche 1 bis 3, wobei die Menge der zugefügten basischen Aminosäure mehr als 15 Gew.% in der Reaktionslösung zum Zeitpunkt der Antigen-Antikörper-Reaktion beträgt oder diesem entspricht.

5. Immunassay gemäß Anspruch 4, wobei die Menge der zugefügten basischen Aminosäure 15 bis einschließlich 17 Gew.% in der Reaktionslösung zum Zeitpunkt der Antigen-Antikörper-Reaktion beträgt.

6. Immunassay gemäß einem der Ansprüche 1 bis 5, wobei die basische Aminosäure Arginin ist.

7. Nachweisreagens für einen Latex-turbidometrischen Immunassay unter Verwendung einer Antigen-Antikörper-Reaktion von Lipoprotein (a) mit Phänotypen, wobei das Reagens folgendes umfaßt: einen Antikörper gegen das Lipoprotein (a) in einer solchen Menge, daß die Menge des Antikörpers mehr als 0,16 mg/ml in einer Reaktionslösung zum Zeitpunkt der Antigen-Antikörper-Reaktion beträgt oder diesem entspricht, und eine basische Aminosäure in einer solchen Menge, daß die Menge der basischen Aminosäure mehr als 15 Gew.% in der Reaktionslösung zum Zeitpunkt der Antigen-Antikörper-Reaktion beträgt oder diesem entspricht, wobei der Latex-turbidometrische Immunassay die Variabilität eines Meßwerts, der einer Phänotypvarietät zuzuschreiben ist, umgeht und ein Meßwert mit einer hohen Korrelation mit einem Meßwert des Lipoproteins (a) in einer biologischen Probe erhalten wird, gemessen auf molekularer Basis.

8. Nachweisreagens für den Latex-turbidometrischen Immunassay gemäß Anspruch 7, wobei die Menge des zugefügten Antikörpers 0,16 bis einschließlich 0,23 mg/ml in der Reaktionslösung zum Zeitpunkt der Antigen-Antikörper-Reaktion beträgt.

9. Nachweisreagens für den Latex-turbidometrischen Immunassay gemäß Anspruch 7 oder 8, wobei die Menge der zugefügten basischen Aminosäure 15 bis einschließlich 17 Gew.% in der Reaktionslösung zum Zeitpunkt der Antigen-Antikörper-Reaktion beträgt.

10. Nachweisreagens für den Latex-turbidometrischen Immunassay gemäß einem der Ansprüche 7 bis 9, wobei die basische Aminosäure Arginin ist.

## Revendications

1. Dosage immunologique turbidimétrique au latex utilisant une réaction antigène-anticorps d'une lipoprotéine (a) ayant plusieurs phénotypes, dans lequel, dans la détection utilisant le dosage immunologique, la quantité d'un anticorps contre la lipoprotéine (a) ajoutée à un système de dosage est ajustée et un acide aminé basique est ajouté au système de dosage, circonvenant ainsi la variabilité d'une valeur de mesure attribuable à la variété de phénotypes et obtenant une valeur de mesure ayant une corrélation élevée avec une valeur de mesure de la lipoprotéine (a) dans un échantillon biologique qui est mesurée sur une base moléculaire.

2. Dosage immunologique selon la revendication 1, dans lequel la quantité de l'anticorps ajoutée est supérieure ou égale à 0,16 mg/mL dans une solution de réaction au moment de la réaction antigène-anticorps.

3. Dosage immunologique selon la revendication 2, dans lequel la quantité de l'anticorps ajoutée est de 0,16 mg/mL à 0,23 mg/mL inclus, dans la solution de réaction au moment de la réaction antigène-anticorps.

4. Dosage immunologique selon l'une quelconque des revendications 1 à 3, dans lequel la quantité de l'acide aminé basique ajoutée est supérieure ou égale à 15 % en poids dans la solution de réaction au moment de la réaction antigène-anticorps.

5. Dosage immunologique selon la revendication 4, dans lequel la quantité de l'acide aminé basique ajoutée est de 15 % en poids à 17 % en poids inclus, dans la solution de réaction au moment de la réaction antigène-anticorps.

6. Dosage immunologique selon l'une quelconque des revendications 1 à 5, dans lequel l'acide aminé basique est l'arginine.

7. Réactif de détection pour un dosage immunologique turbidimétrique au latex utilisant une réaction antigène-anticorps d'une lipoprotéine (a) ayant des phénotypes, le réactif comprenant : un anticorps contre la lipoprotéine (a) dans une quantité telle que la quantité d'anticorps est supérieure ou égale à 0,16 mg/mL dans une solution de réaction au moment de la réaction antigène-anticorps ; et un acide aminé basique dans une quantité telle que la quantité de l'acide aminé basique ajoutée est supérieure ou égale à 15 % en poids dans la solution de réaction au moment de la réaction antigène-anticorps, dans lequel le dosage immunologique turbidimétrique au latex circonvient la variabilité d'une valeur de mesure attribuable à la variété de phénotypes et obtient une valeur de mesure ayant une corrélation élevée avec une valeur de mesure de la lipoprotéine (a) dans un échantillon biologique qui est mesurée sur une base moléculaire.

8. Réactif de détection pour un dosage immunologique turbidimétrique au latex selon la revendication 7, dans lequel la quantité de l'anticorps ajoutée est de 0,16 mg/mL à 0,23 mg/mL inclus, dans la solution de réaction au moment de la réaction antigène-anticorps.

9. Réactif de détection pour un dosage immunologique turbidimétrique au latex selon la revendication 7 ou 8, dans lequel la quantité de l'acide aminé basique ajoutée est de 15 % en poids à 17 % en poids inclus, dans la solution de réaction au moment de la réaction antigène-anticorps.

10. Réactif de détection pour un dosage immunologique turbidimétrique au latex selon l'une quelconque des revendications 7 à 9, dans lequel l'acide aminé basique est l'arginine.
